# EUROPÄISCHE PATENTSCHRIFT

(11) **EP 2 978 888 B1**
(45) Veröffentlichungstag und Bekanntmachung des Hinweises auf die Patenterteilung: **12.04.2017**
(21) Anmeldenummer: 14706643.5
(22) Anmeldetag: 26.02.2014
(51) Int. Cl.: D06F 35/00, A47L 15/42, A21B 3/00, A47J 17/02, A47J 19/02, A47J 31/00

(54) **HAUSGERÄT MIT EINER KATALYTISCH WIRKSAMEN OBERFLÄCHE SOWIE VERFAHREN ZU SEINEM BETRIEB**
HOUSEHOLD APPLIANCE HAVING A CATALYTICALLY EFFECTIVE SURFACE AND METHOD FOR THE OPERATION THEREOF
APPAREIL MÉNAGER AYANT UNE SURFACE CATALYTIQUEMENT ACTIVE AINSI QUE PROCÉDÉ POUR LE FAIRE FONCTIONNER

(30) Priorität: 26.03.2013 DE 102013205302
(43) Veröffentlichungstag der Anmeldung: 03.02.2016
(73) Patentinhaber: BSH Hausgeräte GmbH, 81739 München (DE)
(72) Erfinder: HANAU, Andreas, 12359 Berlin (DE); BARRADO FRANCO, Antonio, 10317 Berlin (DE); BISCHOF, Andreas, 10407 Berlin (DE); SCHAUB, Hartmut, 14656 Brieselang (DE)
(86) Internationale Anmeldenummer: PCT/EP2014/053751
(87) Internationale Veröffentlichungsnummer: WO 2014/154432

(56) Entgegenhaltungen:
- EP-A2- 0 761 809
- DE-A1-102009 026 712
- KR-A- 20070 066 334

## Beschreibung

Die Erfindung betrifft ein Hausgerät, welches in einer Oberfläche mindestens eine katalytisch wirksame Substanz enthält, sowie ein bevorzugtes Verfahren zu seinem Betrieb (siehe KR-A-20070066334).

Hausgeräte können mit Staub, Schmutz, Lebensmitteln, Feuchtigkeit oder Lebewesen wie Menschen oder Tieren in Kontakt kommen. Dies kann Hygieneprobleme ergeben, da sich Mikroorganismen auf den Hausgeräten anlagern und vermehren können. Insbesondere kann es zu verunreinigten, nicht mehr hygienisch einwandfreien Hausgeräten führen oder aber zumindest zu Hausgeräten, welche unansehnliche Verfärbungen aufweisen. Im schlimmsten Fall kann dies gesundheitsschädlich sein. Eine regelmäßige Reinigung von Hausgeräten ist daher sinnvoll, wobei allerdings Einfachheit und Wirksamkeit einer solchen Reinigung von Art und Menge der Verunreinigung mit Mikroorganismen abhängen können. Unter Umständen kann sich ein Biofilm ausbilden oder bereits vorliegen. Biofilme, welche aus organischen Substanzen wie Mikroorganismen und Nährstoffen bestehen, führen zu Geruchsbeeinträchtigungen und/oder sichtbaren Verunreinigungen.

Dies ist insbesondere bei Hausgeräten von Bedeutung, bei welchen innere und/oder äußere Oberflächen in Kontakt mit Lebensmitteln kommen, die im Hausgerät beispielsweise aufbewahrt, verarbeitet oder zubereitet werden.

Andere Hausgeräte, welche zu Verunreinigungen mit Mikroorganismen neigen können, sind aber auch beispielsweise Dunstabzugshauben und Staubsauger.

In wasserführenden Hausgeräten werden im Allgemeinen Gegenstände, die auf unterschiedliche Weise verunreinigt sind, gereinigt. So fallen in Geschirrspülmaschinen Essensreste an und das Spektrum an Verunreinigungen bei in Waschmaschinen zu reinigenden Wäschestücken ist in der Regel noch größer. Allen wasserführenden Hausgeräten ist gemeinsam, dass in der feuchtwarmen Atmosphäre, insbesondere an weniger gut zugänglichen Stellen, Verunreinigungen entstehen und sich anreichern können. Diese Verunreinigungen können ein guter Nährboden für Mikroorganismen wie Bakterien oder Pilze sein.

Unabhängig von der Art des Hausgerätes ergibt sich im Allgemeinen das Problem, dass zur Bedienung eine Bedieneinheit des Hausgerätes in der Regel von einem Benutzer berührt werden muss. Dadurch können insbesondere Bedieneinheiten mit Mikroorganismen verunreinigt sein, die außerdem bei einer Benutzung des Hausgerätes durch unterschiedliche Benutzer leicht weitergegeben werden können.

Ein weiteres Problem besteht darin, dass sich Mikroorganismen in einer feuchten Atmosphäre leichter und rascher ausbreiten. Dies ist insbesondere bei schlecht belüfteten Bereichen der Fall, die zudem häufig schwer zugänglich sind. Beispielsweise kann bei einem Kühlgerät das Auftreten von Kondenswasser das Wachstum von Mikroorganismen fördern. Aber auch Speicher für Flüssigkeiten wie Wasser und Milch, beispielsweise in Kaffeemaschinen sind Bereiche, die prinzipiell anfällig für das Wachstum von Mikroorganismen sind.

Wünschenswert wäre es daher, ein Hausgerät bereit zu stellen, das leicht und effizient gereinigt werden kann und/oder bei dem die Ausbildung von Biofilmen möglichst verhindert ist.

Zur Beseitigung und/oder Vermeidung von Biofilmen sind verschiedene Maßnahmen bekannt. Insbesondere werden Gerätereinigungsprogramme angeboten, die in wasserführenden Hausgeräten bei hohen Temperaturen mit Unterstützung von Waschmitteln und teilweise unter erhöhten Flottenständen und/oder bei höheren Trommeldrehzahlen, d.h. bei erhöhtem Eintrag mechanischer Energie, entstandene Verschmutzungen wieder beseitigen. Bekannt ist außerdem die Verwendung von Ozon zur Entfernung von organischen Verunreinigungen.

Ebenso wurde bereits vorgeschlagen, mit Hilfe von UV-C-Strahlern im Durchlaufprinzip organische Verschmutzungen zu beseitigen, wobei die Beseitigung in der Weise erfolgt, dass Mikroorganismen in solchen Verschmutzungen letztlich durch Schädigung ihrer Erbsubstanzen abgetötet werden. Es sind ebenfalls photokatalytische Methoden bekannt, beispielsweise der Einsatz von Titandioxidbeschichtungen für die Desodorierung, Desinfizierung und Reinigung. Hierbei ist eine Aktivierung des Katalysators durch UV-Strahlung nötig.

Bekannt sind ebenfalls Maßnahmen zur Abtötung von Mikroorganismen unter Verwendung von Ag⁺- oder Cu⁺-Ionen in einer Waschflotte bzw. auf den Oberflächen von mit der Waschflotte in Berührung stehenden Materialien.

Andere Verfahren zielen auf die thermische Abtötung eventuell vorhandener Mikroorganismen durch Erhöhung der Temperatur an den Oberflächen der Bauteile über eine direkte oder indirekte Energieübertragung (Wasser, Dampf, Mikrowelle).

Nachteilig bei diesen bekannten Verfahren und Maßnahmen sind der hohe Energieaufwand sowie die zur Erzielung ausreichend großer Effekte unter Umständen hohen Apparate- und/oder Betriebskosten. Beim Einsatz von Ag⁺- oder Cu⁺-Ionen kommen nachteilige Auswirkungen hinsichtlich Boden- und Gewässerbelastung hinzu. Bei einigen Methoden werden aggressive Mittel verwendet, z.B. Ozon oder UV-Strahlung, so dass zusätzliche Sicherheitsmaßnahmen erforderlich sein können.

Polyoxometallaten werden auf unterschiedlichen Gebieten eingesetzt, beispielsweise in der Analytischen und Klinischen Chemie, in der Katalyse (einschließlich Photokatalyse), in der Biochemie (Inhibierung von Elektronentransferprozessen), in der Medizin (antitumorale und antivirale Aktivität) sowie bei der Herstellung integrierter Schaltkreise. Als Oxidationskatalysatoren sind Polyoxometallate vor allem in der Papier- und Kunststoffindustrie bekannt.

Im Haushalt ist die Anwendung von Polyoxometallaten als Bleichekatalysatoren bekannt. Beim Bleichen wird die Struktur eines Farbstoffs durch ein starkes Oxidationsmittel (Bleichmittel) zerstört. Bekannt sind vor allem Bleichmittel auf Sauerstoffbasis wie Peroxide und Bleichmittel auf Chlorbasis. Um die Wirkung der relativ milden Bleichmittel auf Sauerstoffbasis, vor allem bei Waschtemperaturen bis 60°C, zu verstärken, ist der Einsatz von Bleichaktivatoren und/oder Bleichkatalysatoren bekannt.

Die EP 0 761 809 B1 beschreibt eine Bleichmittelzusammensetzung, die Polyoxometallate als Bleichmittelkatalysator enthält. Dabei steigern Polyoxometallate bereits in geringen Mengen die Wirksamkeit von Bleichmitteln wie Wasserstoffperoxid, anorganischen und organischen Persäuren bzw. Caroaten. Die beschriebene Bleichmittelzusammensetzung umfasst Bleichmittel (Peroxid) und Bleichmittelkatalysatoren (Polyoxometallate) und kann beispielsweise in Waschmitteln, Reinigungsmitteln, Desinfektionsmitteln und Gebissreinigern verwendet werden.

Die EP 1 141 210 B1 beschreibt ein Verfahren zum Bleichen von Wäsche oder Haushaltsoberflächen, bei dem ein Waschmedium, das Polyoxometallate enthält, mit dem verschmutzten Substrat in Kontakt gebracht wird. Dabei dient Luft als primäre Quelle von Sauerstoffatomen zum Bleichen.

Die WO 2005/059226 A1 beschreibt ein Verfahren zum Wäschewaschen in einer Waschmaschine, wobei die lonenstärke der Waschlauge während eines Waschganges geändert wird, um die Reinigungsfähigkeit der Waschlauge zu optimieren. Dabei kann die Waschmittellauge ein Bleichmittelsystem enthalten. Als bleichende Oxidationskatalysatoren mit Peroxybleichmitteln und Luft werden in diesem Zusammenhang anorganische Polyoxometallate beschrieben.

Die DE 10 2009 026 712 A1 beschreibt ein Hausgerät mit mindestens einem Bauteil, welches eine durch eine organische Verschmutzung belastbare Oberfläche aufweist, wobei die Oberfläche einen Photokatalysator aufweist. Dieser Oberfläche ist eine Photoquelle zum Bestrahlen des Photokatalysators mit einer aktivierenden elektromagnetischen Strahlung zugeordnet, wobei die Oberfläche von einem urgeformten ersten Werkstoff gebildet ist, in welchem der Photokatalysator dispergiert ist. Als Photokatalysator sind im Detail Materialien mit Titandioxid und dessen Modifikationen beschrieben.

Aufgabe der vorliegenden Erfindung war es vor diesem Hintergrund, ein Hausgerät sowie ein Verfahren zu seinem Betrieb bereitzustellen, bei dem auf einfache und besonders hygienische Weise Verunreinigungen beseitigt oder verhindert oder zumindest weitgehend beseitigt oder verhindert werden können. Vorzugsweise sollen Verunreinigungen mit Mikroorganismen beseitigt oder verhindert werden.

Die Lösung dieser Aufgabe wird erfindungsgemäß erreicht durch ein Hausgerät gemäß Anspruch 1.

Bevorzugte Ausführungsformen des erfindungsgemäßen Hausgeräts sind in den entsprechenden abhängigen Patentansprüchen aufgeführt.

Gegenstand der Erfindung ist somit ein Hausgerät, welches in einer Oberfläche mindestens eine katalytisch wirksame Substanz enthält, wobei die katalytisch wirksame Substanz ein Polyoxometallat ist, welches in einer inneren und/oder äußeren Oberfläche des Hausgerätes enthalten ist, mit der Maßgabe, dass das Polyoxometallat zumindest in einer äußeren Oberfläche des Hausgerätes enthalten ist, wenn das Hausgerät ein wasserführendes Hausgerät mit einem Behälter zur Aufnahme von zu reinigenden Gegenständen ist.

Eine "äußere Oberfläche" des Hausgerätes ist hierbei im Allgemeinen eine Oberfläche, die bei einem üblichen Betrieb des Hausgerätes für einen Benutzer des Hausgerätes zugänglich ist, ohne den Betrieb zu stören. Dies bedeutet im Allgemeinen, dass eine äußere Oberfläche des Hausgerätes ohne Störung des Betriebes des Hausgerätes berührt und betrachtet werden kann. Eine "innere Oberfläche" des Hausgerätes ist im Gegensatz hierzu im Allgemeinen eine Oberfläche, die bei einem üblichen Betrieb des Hausgerätes für einen Benutzer des Hausgerätes ohne Störung des Betriebes nicht zugänglich ist. "Innere Oberflächen" im Sinne der Erfindung sind daher beispielsweise auch die innere Oberfläche eines Wasserbehälters einer Kaffeemaschine oder das Innere eines Kühlgerätes. "Äußere Oberflächen" sind daher insbesondere Bedienelemente sowie das Gehäuse des Hausgerätes.

Polyoxometallate sind anorganische Metall-Sauerstoff-Cluster. Sie besitzen im Allgemeinen polyatomische Anionen, die aus drei oder mehr Übergangsmetall-Oxyanionen, insbesondere Wolframat, Molybdat, Vanadat, Niobat und/oder Tantalat, aufgebaut und über Sauerstoffatome verbrückt sind. Polyoxometallate können eine große dreidimensionale Netzwerkstruktur mit definierten oligomeren oder polymeren Struktureinheiten bilden.

Ihrer Struktur entsprechend werden die Polyoxometallate in Iso- und Heteropolyoxometallate unterteilt. Isopolyoxometallate sind die einfachsten Formen von Polyoxometallaten und lassen sich als binäre, d. h. nur Metallion und Sauerstoff enthaltende Oxid-Anionen beschreiben. Typische Beispiele für derartige Isopolyoxometallate sind [Mo₂O₇]²⁻, [W₆O₂₄]¹²⁻, [Mo₆O₁₆]²⁻, [Mo₃₆O₁₁₂]⁸⁻. Im Unterschied hierzu enthalten Heteropolyoxometallate, die in großem Umfang als Oxidationskatalysatoren verwendet werden, noch weitere Nichtmetall-, Halbmetall-und/oder Übergangsmetallionen. So sind z. B. übergangsmetalldotierte, sogenannte Keggin-Anionen der Formel [APW₁₁O₃₉]^{7-/8-} mit A = Zn, Co, Ni, Mn und Dawson-Anionen [AP₂W₁₇O₆₁]^{7-/8-} mit A = Mn, Fe, Co, Ni, Cu bekannt, die zusätzlich auch Kristallwasser gebunden haben können. Weitere Substitutionen, auch unterschiedlicher Übergangsmetallionen, sind bekannt, z. B. [WZnMn₂(ZnW₉O₃₄)₂]¹²⁻ oder [SiV₃W₉O₄₀]⁷⁻. Der Ladungsausgleich der vorstehend beschriebenen Anionen erfolgt entweder über Protonen, wobei die entsprechenden Polysäuren erhalten werden, oder über Kationen unter Bildung der Salze der Polysäuren (Heteropolyoxometallate).

Der hierin verwendete Begriff Polyoxometallat umfasst sowohl die Salze der Polysäuren als auch die entsprechenden Polysäuren. Zu den erfindungsgemäß verwendeten Polyoxometallaten zählen die vorstehend genannten Polyoxometallate und beispielsweise die in der EP 0761 809 B1 und EP 1 141 210 B1 beschriebenen Polyoxometallate.

Bevorzugt ist das Polyoxometallat ein Wolframat. Hierbei kann es sich um ein Isopolywolframat oder ein Heteropolywolframat handeln. Besonders bevorzugt wird ein Titan-modifiziertes Wolframat oder ein Vanadium-modifiziertes Wolframat eingesetzt. Erfindungsgemäß ist es insbesondere bevorzugt, dass das Wolframat Vanadium enthält. Hierbei umfasst das Wolframat vorzugsweise das [SiV₃W₉O₄₀]⁷⁻-Anion.

Die Polyoxometallate liegen erfindungsgemäß bevorzugt als Salze vor, die aus einem geeigneten Kation und einem Polyoxometallat-Anion bestehen oder dieses umfassen. Geeignete Katione sind beispielsweise Tetraalkylammoniumkationen. Durch geeignete Wahl von Anion und Kation können Polyoxometallate zur Verfügung gestellt werden, welche als Beschichtung einer inneren und/oder äußeren Oberfläche des Hausgerätes verwendet werden können. Vorzugsweise liegt das Polyoxymetallat zumindest teilweise als Tetraalkylammoniumsalz vor.

In einer bevorzugten Ausführungsform des Hausgerätes umfasst die mindestens eine Polyoxymetallat enthaltende äußere Oberfläche ein Bedienungselement des Hausgeräts. Bedienungselemente sind insbesondere durch Druck, Annäherung oder Berührung zu bedienende Elemente, mit welchen der Betrieb des Hausgerätes im Allgemeinen gesteuert wird. Bedienungselemente sind insbesondere Tasten, Schalter, Kipphebel, Drehwahlschalter, Schieb- und Drehregler, berührungsempfindliche Displays wie insbesondere Touchscreens, beispielsweise kapazitive Touchscreens.

Zu den Bedienungseinheiten im Weiteren Sinn gehören insbesondere auch Türgriffe z.B. eines wasserführenden Hausgerätes, eines Kühlgerätes oder eines Ofens oder sonstige Griffe, welche zur Benutzung eines Hausgerätes erforderlich sind, z.B. der Griff eines Staubsaugers oder Mixgerätes.

In einer weiteren bevorzugten Ausführungsform des Hausgerätes ist eine Polyoxymetallat enthaltende innere Oberfläche eine innere Wand eines Wasserbehälters und/oder eines Durchströmungselementes des Hausgerätes.

Der Wasserbehälter kann beispielsweise der Wasserbehälter einer Kaffeemaschine oder eines Kaffeeautomaten sein.

Unter Durchströmungselementen werden im Allgemeinen Bauteile verstanden, die im Strömungsfluss einer wässrigen Flüssigkeit oder eines Gases im Hausgerät angebracht sind. Dabei handelt es sich vorzugsweise um gitter- oder filterartige Strukturen. Dabei können erfindungsgemäß bereits im Hausgerät zu anderen Zwecken vorhandene Durchströmungselemente mit einer Polyoxometallat enthaltenden inneren Oberfläche versehen werden oder zusätzliche, Polyoxometallat auf einer inneren Oberfläche enthaltende Durchströmungselemente im Hausgerät angeordnet werden. Durch einfache Modifizierungen kann eine Polyoxometallat enthaltende innere Oberfläche bei Durchströmungselementen weiter vergrößert werden und es können für eine Oxidationsreaktion an Polyoxometallaten vorteilhafte Turbulenzen im strömenden flüssigen oder gasförmigen Medium erzeugt werden.

Ein Durchströmungselement des Hausgerätes ist insbesondere ein Element, durch das ein gasförmiges oder flüssiges Medium strömt und bei dem durch Strömen eventuell auf einer inneren Wand haftende Mikroorganismen oder sonstige Bestandteile eines Biofilmes mitgerissen werden können. Andererseits können im gasförmigen oder flüssigen Medium Mikroorganismen oder das Wachstum von Mikroorganismen fördernde Bestandteile vorhanden sein. Durchströmungselemente im Sinne der Erfindung sind somit insbesondere Flüssigkeitsleitungen in einer Kaffeemaschine, die luftführenden Kanäle in einer Dunstabzugshaube oder die luftführenden Kanäle in einem Staubsauger.

Ein erfindungsgemäßes Hausgerät umfasst in einer bevorzugten Ausführungsform ein Behältnis, in welchem ein Lebensmittel aufbewahrt, verarbeitet oder zubereitet wird. "Aufbewahren" bedeuten hierbei insbesondere ein Aufbewahren in einer schützenden Umgebung, beispielsweise unter das Verderben der Lebensmittel verlangsamenden oder verhindernden kühlen oder kalten Umgebungsbedingungen. "Verarbeiten" bedeutet insbesondere ein Schneiden, Zerhacken oder Auspressen von Lebensmitteln wie Gemüse und Obst. "Zubereiten" bedeutet beispielsweise ein Kochen, Extrahieren, Gären und Braten eines Lebensmittels.

In einer bevorzugten Ausführungsform ist das Hausgerät ausgewählt aus der Gruppe, die aus einem Kühlgerät, einer Kaffeemaschine, einem Ofen, einem Mikrowellengerät, einem Gemüseschneider und einem Entsafter besteht.

In Kaffeemaschinen und Kaffeeautomaten ist die Erfindung insbesondere bedeutsam für Wasserspeicher, Wasserleitungen, Siebträger, Tropfschalen, Milchschäumer und Tresterbehälter. Daneben ist die Erfindung bedeutsam für Wasserkocher, Teebereiter und Heißwasserspender.

In Kühlgeräten, zu denen hierin umfassend auch Gefriergeräte gehören, kann die Erfindung zur Hygienisierung der inneren Oberflächen und des auf den inneren Oberflächen stehenden Kondenswassers verwendet werden sowie im Kondenswasserbehälter und Zuleitungen.

In einem anderen bevorzugten Aspekt ist das hierin beanspruchte Hausgerät ein wasserführendes Hausgerät mit einem Behälter zur Aufnahme von zu behandelnden Gegenständen, das ausgewählt ist aus der Gruppe, die aus einer Waschmaschine, einem Waschtrockner, einem Trockner und einem Geschirrspülgerät besteht. Im Sinne der vorliegenden Erfindung weist ein solches wasserführendes Hausgerät mindestens eine äußere Oberfläche auf, die ein Polyoxymetallat enthält, z.B. auf einer Bedienungseinheit oder einem Gehäuseteil.

Das erfindungsgemäße Hausgerät kann auch ein Staubsauger und Bodenpflegegerät sein, wo die Polyoxometallat enthaltenden Oberflächen insbesondere im Bereich der Filter und Vliese und des Staubbeutels sowie der Staubbeutelaufnahme sind.

Überdies kann das erfindungsgemäße Hausgerät auch ein Ofen, ein Mikrowellengerät oder ein Dampfgarer, ein Herd oder eine Kochmulde sein, wobei die Oberflächen aufgrund eines Gehaltes an Polyoxometallat hygienisiert sein können.

Weitere Beispiele für erfindungsgemäße Hausgeräte sind Durchlauferhitzer; Dampfstationen und Dampfbügeleisen; Luftbefeuchter, Klimaanlagen und Klimageräte. Hier ist sowohl die Hygienisierung der Oberflächen als auch die des zu erhitzenden Wassers von Bedeutung. Generell ist es auch hier wichtig, dass durch die Hygienisierung der Oberflächen bei Hautkontakt die Übertragung von möglicherweise krankheitserregenden Mikroorganismen oder Pilzen vermieden werden kann.

Bei Dunstabzugshauben als einem weiteren Beispiel für ein erfindungsgemäßes Hausgerät sind die Hygienisierung der Oberflächen, insbesondere der Siebe und der Filtermatten, sowie auch die desinfizierende Behandlung der Luft von Bedeutung.

Das im Sinne der Erfindung eingesetzte Polyoxymetallat kann auf unterschiedliche Art und Weise eingesetzt werden, insbesondere in Form von Partikeln, als Beschichtung oder als Teil einer Beschichtung.

Die Polyoxometallat enthaltende Oberfläche kann auf beliebige Weise erzeugt werden, solange die erfindungsgemäße katalytische Wirkung möglich ist. So kann sie beispielsweise durch Bildung eines Polyoxometallat enthaltenden Filmes erzeugt werden oder aber z.B. durch Platzierung von Polyoxometallat-Partikeln an der Oberfläche eines porösen Materials. Die Erzeugung wird insbesondere von Ort und Art der Anwendung abhängen.

In einer bevorzugten Ausführungsform der Erfindung, die sich insbesondere für die Verwendung in Hausgeräten eignet, in welchen flüssige oder gasförmige Medien, die Mikroorganismen oder deren Wachstum fördernde Substanzen enthalten, gereinigt werden sollen, ist im Hausgerät die mindestens eine innere Oberfläche gebildet durch Partikel, welche mindestens ein Polyoxymetallat enthaltendes Salz enthalten oder aus diesem bestehen, wobei die Partikel in einer Filtriereinheit platziert sind, welche für ein gasförmiges oder flüssiges Medium durchlässig ist. Die Filtriereinheit ist dabei erfindungsgemäß nicht beschränkt, solange der Zweck der Filtrierung erreicht werden kann. Es kann sich somit um einen porösen rigiden Behälter aber auch um eine durchlässige Tasche oder Umhüllung aus einem Gewebe handeln.

Die Erfindung ermöglicht, insbesondere wenn die Wirkungsweise von Polyoxometallat durch das Inkontaktbringen mit einem Behandlungsmittel enthaltend ein Oxidationsmittel, beispielsweise in Form einer wässrigen Lösung des Oxidationsmittels, verstärkt wird, dass organische Substanzen wie Mikroorganismen und Nährstoffe abgebaut werden können und somit der Ausbildung eines Biofilms im Hausgerät entgegengewirkt werden kann. Dabei wird insbesondere die Eigenschaft von Polyoxometallaten, als Oxidationskatalysator zu wirken, ausgenutzt. An der Polyoxometallat enthaltenden inneren oder äußeren Oberfläche des Hausgeräts bilden sich im Allgemeinen in Gegenwart eines Oxidationsmittels, wie beispielsweise Sauerstoff, Wasserstoffperoxid oder Ozon, Sauerstoffradikale (oder Sauerstoff enthaltende Radikale, im Folgenden zusammenfassend als "Sauerstoffradikale" bezeichnet). Aufgrund ihrer chemischen Reaktivität können diese Sauerstoffradikale insbesondere organische Verbindungen zersetzen und sind folglich schädlich für Mikroorganismen. Auf diese Weise werden organische Substanzen beispielsweise in Wasser, das mit einer mit Polyoxometallat beschichteten inneren und/oder äußeren Fläche des Hausgeräts in Kontakt gebracht wird, abgebaut und somit einem Biofilm entgegen gewirkt.

Bei der Oxidationsreaktion dient somit das Polyoxometallat im Allgemeinen als Oxidationskatalysator, der zusammen mit einem Oxidationsmittel wirkt. Das Oxidationsmittel ist erfindungsgemäß nicht eingeschränkt. Vorzugsweise werden Sauerstoff enthaltende Oxidationsmittel eingesetzt. Hierbei sind Sauerstoff, anorganische oder organische Peroxide und/oder Ozon als Oxidationsmittel besonders bevorzugt. Unter diesen ist wiederum Sauerstoff als Oxidationsmittel besonders bevorzugt, da hierdurch ein zusätzlicher Eintrag von möglicherweise schädlichen oder störenden Substanzen vermieden werden kann. Als Quelle für Sauerstoff dient dabei insbesondere Luft.

Ozon ist als Oxidationsmittel in Hausgeräten mit einem Ozongenerator bevorzugt. Derartige Hausgeräte verfügen vorzugsweise über eine Ozonbeseitigungsvorrichtung für die Beseitigung von überschüssigem Ozon.

Um eine fortlaufende Oxidationsreaktion zu ermöglichen, sollte eine ausreichende Mobilität der oxidierenden Spezies, z.B. von Sauerstoffradikalen, die an der als Oxidationskatalysator wirkenden Polyoxometallat enthaltenden inneren Oberfläche erzeugt wurden, gegeben sein. Das Maß an gewünschter Mobilität hängt im Allgemeinen vom Verwendungszweck ab, ob also beispielsweise ein Bedienungselement an sich hygienisch ausgestattet werden soll oder ob Mikroorganismen auch in einem umgebenden Medium bekämpft werden sollen.

Vorzugsweise weist das erfindungsgemäße Hausgerät daher ein der Filtriereinheit zugeordnetes Umwälzelement und/oder Lufteinleitungselement auf. Damit wird die Möglichkeit der Wechselwirkung mit einer katalysierend wirkenden Polyoxometallat enthaltenden Oberfläche erhöht. Außerdem verbessert sich die Möglichkeit für die Zufuhr eines Oxidationsmittels an die Polyoxometallat enthaltende Oberfläche. Auf diese Weise wird eine ausreichende Mobilität der an der Katalysatoroberfläche erzeugten oxidierenden Spezies, z.B. Sauerstoffradikale, gewährleistet, um eine fortlaufende Reaktion zu ermöglichen. Außerdem wird hierbei eine genügend hohe Sauerstoffkonzentration ermöglicht, um eine möglichst vollständige Katalysatorreaktion bereit zu stellen.

In einer weiteren bevorzugten Ausführungsform des Hausgeräts umfasst dieses daher ein Mittel zur Erzeugung oder Einleitung eines Oxidationsmittels, dessen oxidierende Wirkung auf Mikroorganismen durch Wechselwirkung mit der ein Polyoxymetallat enthaltenden inneren oder äußeren Oberfläche des Hausgerätes verstärkt wird. Vorzugsweise ist hierbei das Oxidationsmittel Sauerstoff, Ozon oder ein Peroxid. Es ist erfindungsgemäß bevorzugt, dass die Polyoxometallat enthaltende innere oder äußere Oberfläche des Hausgeräts eine Dicke von nicht mehr als 50 µm und besonders bevorzugt eine Dicke von nicht mehr als 10 µm hat.

An einer Polyoxometallat enthaltenden inneren oder äußeren Oberfläche des Hausgeräts bilden sich in Gegenwart eines Oxidationsmittels, das beispielsweise im Wasser gelöst oder in Wasser oder Luft vermischt vorliegen kann, z.B. Sauerstoffradikale. Kommen diese sehr reaktiven Sauerstoffradikale in Kontakt mit organischen Substanzen, einschließlich Mikroorganismen, läuft eine Oxidationsreaktion ab, die zum Abbau der organischen Substanzen bzw. zu pathologischen Prozessen bei den Mikroorganismen führt. Auf diese Weise wird der Bildung eines Biofilms entgegen gewirkt.

Beispielsweise wird wie bereits erwähnt ein Sauerstoff enthaltendes Oxidationsmittel verwendet, insbesondere Sauerstoff, wobei Luft als Quelle für den Sauerstoff dient. Dabei kann der Sauerstoff beispielsweise im Wasser gelöst vorliegen. Außerdem kann es durch eine entsprechende Bewegung von Sauerstoff enthaltender Luft oder von Sauerstoff enthaltendem Wasser zu einer Vermischung von Luft und Wasser kommen, beispielsweise zur Ausbildung von Luftbläschen im Wasser. Ein solches Luft-Wasser-Gemisch kann in Kontakt mit der Polyoxometallat enthaltenden Oberfläche gebracht werden, wobei der Luftsauerstoff als Oxidationsmittel dient.

Beispielsweise wird ein Peroxid oder Ozon als Oxidationsmittel verwendet. Ozon kann vorteilhaft bei Geräten mit Ozongenerator als Oxidationsmittel benutzt werden. Die Polyoxometallat enthaltende Oberfläche ist dann so angeordnet, dass sie in Kontakt mit Ozon kommen kann.

Die Erfindung hat den Vorteil, dass auf einfache und kostengünstige Weise ein Hausgerät zur Verfügung gestellt wird, das deutlich weniger zu Verunreinigungen, insbesondere auch mit Mikroorganismen, neigt und für den Fall dennoch auftretender Verunreinigungen leicht zu reinigen ist.

Dabei liegt ein besonderer Vorteil der Verwendung von Polyoxometallat enthaltenden inneren und äußeren Oberflächen darin, dass diese über Jahre hinweg als katalytisches System funktionieren können, ohne dass eine zusätzliche Aktivierung oder ein zusätzlicher Energieverbrauch nötig wäre. Nur bei Verwendung eines zusätzlichen Umwälz- oder Lufteinleitungselements würde ein zusätzlicher Energieverbrauch stattfinden, wobei dieser im Vergleich zu anderen Reinigungsverfahren vergleichsweise gering ausfiele. Außerdem müssen keine zusätzlichen Reagenzien hinzu dosiert werden und auch eine Aktivierung des Katalysators, beispielsweise durch UV-Strahlung, entfällt. Außerdem können Polyoxometallate viruzide Wirkungen haben. Somit wird durch die vorliegende Erfindung ein Hygienesystem für Hausgeräte nahezu ohne laufende Kosten und mit langer Lebensdauer bereitgestellt.

Das Reinigen ist aufgrund der besonderen Oberflächen weniger aufwendig. Insbesondere kann ein Reinigen weniger häufig oder gar nicht mehr erforderlich sein. Überdies ermöglicht die Erfindung auch eine Fernwirkung in dem Sinn, dass nicht nur Polyoxometallat enthaltende Oberflächen geschützt oder leicht von Mikroorganismen befreit werden können. Die an Polyoxometallat enthaltenden Oberflächen stattfindenden Oxidationsreaktionen können zusätzlich auch benachbarte Bereiche in einem Hausgerät beeinflussen und dort Mikroorganismen bekämpfen.

## Patentansprüche

1. Hausgerät, welches in einer Oberfläche mindestens eine katalytisch wirksame Substanz enthält, **dadurch gekennzeichnet, dass** die katalytisch wirksame Substanz ein Polyoxometallat ist, welches in einer inneren und/oder äußeren Oberfläche des Hausgerätes enthalten ist, mit der Maßgabe, dass das Polyoxometallat zumindest in einer äußeren Oberfläche des Hausgerätes enthalten ist, wenn das Hausgerät ein wasserführendes Hausgerät mit einem Behälter zur Aufnahme von zu reinigenden Gegenständen ist, wobei eine äußere Oberfläche des Hausgerätes eine Oberfläche ist, die bei einem üblichen Betrieb des Hausgerätes für einen Benutzer des Hausgerätes zugänglich ist, ohne den Betrieb zu stören, und wobei eine innere Oberfläche des Hausgerätes eine Oberfläche ist, die bei einem üblichen Betrieb des Hausgerätes für einen Benutzer des Hausgerätes ohne Störung des Betriebs nicht zugänglich ist.

2. Hausgerät nach Anspruch 1, **dadurch gekennzeichnet, dass** das Polyoxometallat ein Wolframat ist.

3. Hausgerät nach Anspruch 2, **dadurch gekennzeichnet, dass** das Wolframat Vanadium enthält.

4. Hausgerät nach Anspruch 3, **dadurch gekennzeichnet, dass** das Wolframat das [SiV₃W₉O₄₀]⁷⁻-Anion umfasst.

5. Hausgerät nach Anspruch 1 oder 2, **dadurch gekennzeichnet, dass** das Wolframat Titan-modifiziert ist.

6. Hausgerät nach einem der Ansprüche 1 bis 5, **dadurch gekennzeichnet, dass** mindestens eine Polyoxymetallat enthaltende äußere Oberfläche ein Bedienungselement des Hausgeräts umfasst.

7. Hausgerät nach einem der Ansprüche 1 bis 6, **dadurch gekennzeichnet, dass** eine Polyoxymetallat enthaltende innere Oberfläche eine innere Wand eines Wasserbehälters und/oder eines Durchströmungselementes des Hausgerätes ist.

8. Hausgerät nach einem der Ansprüche 1 bis 7, **dadurch gekennzeichnet, dass** das Hausgerät ein Behältnis umfasst, in welchem ein Lebensmittel aufbewahrt, verarbeitet oder zubereitet wird.

9. Hausgerät nach Anspruch 8, **dadurch gekennzeichnet, dass** das Hausgerät ausgewählt ist aus der Gruppe, die aus einem Kühlgerät, einer Kaffeemaschine, einem Ofen, einem Mikrowellengerät, einem Gemüseschneider und einem Entsafter besteht.

10. Hausgerät nach einem der Ansprüche 1 bis 7, **dadurch gekennzeichnet, dass** das Hausgerät ein wasserführendes Hausgerät ist mit einem Behälter zur Aufnahme von zu behandelnden Gegenständen, das ausgewählt ist aus der Gruppe, die aus einer Waschmaschine, einem Waschtrockner, einem Trockner und einem Geschirrspülgerät besteht.

11. Hausgerät nach einem der Ansprüche 1 bis 10, **dadurch gekennzeichnet, dass** die mindestens eine innere Oberfläche gebildet ist durch Partikel, welche mindestens ein Polyoxymetallat enthaltendes Salz enthalten oder aus diesem bestehen, wobei die Partikel in einer Filtriereinheit platziert sind, welche für ein gasförmiges oder flüssiges Medium durchlässig ist.

12. Hausgerät nach Anspruch 11, **dadurch gekennzeichnet, dass** das Hausgerät ein der Filtriereinheit zugeordnetes Umwälzelement und/oder Lufteinleitungselement aufweist.

13. Hausgerät nach einem der Ansprüche 1 bis 12, umfassend ein Mittel zur Erzeugung oder Einleitung eines Oxidationsmittels, dessen oxidierende Wirkung auf Mikroorganismen durch Wechselwirkung mit der ein Polyoxymetallat enthaltenden inneren oder äußeren Oberfläche des Hausgerätes verstärkt wird.

14. Hausgerät nach Anspruch 13, **dadurch gekennzeichnet, dass** das Oxidationsmittel Sauerstoff, Ozon oder ein Peroxid ist.

15. Hausgerät nach einem der Ansprüche 1 bis 14, **dadurch gekennzeichnet, dass** das Polyoxymetallat zumindest teilweise als Tetraalkylammoniumsalz vorliegt.

## Claims

1. Household appliance, which contains at least one catalytically effective substance in a surface, **characterised in that** the catalytically effective substance is a polyoxometalate, which is contained in an inner and/or outer surface of the household appliance, provided that the polyoxometalate is contained at least in an outer surface of the household appliance, if the household appliance is a water-bearing household appliance with a container for receiving objects to be cleaned, wherein an outer surface of the household appliance is a surface which is accessible to a user of the household appliance during conventional operation of the household appliance without interfering with operation, and wherein an inner surface of the household appliance is a surface which is not accessible to a user of the household appliance during conventional operation of the household appliance without interfering with operation.

2. Household appliance according to claim 1, **characterised in that** the polyoxometalate is wolframate.

3. Household appliance according to claim 2, **characterised in that** the wolframate contains vanadium.

4. Household appliance according to claim 3, **characterised in that** the wolframate includes the [SiV₃W₉O₄₀]⁷⁻ anion.

5. Household appliance according to claim 1 or 2, **characterised in that** the wolframate is titanium-modified.

6. Household appliance according to one of claims 1 to 5, **characterised in that** at least one outer surface containing polyoxymetalate includes a control element of the household appliance.

7. Household appliance according to one of claims 1 to 6, **characterised in that** an inner surface containing polyoxometalate is an inner wall of a water container and/or a throughflow element of the household appliance.

8. Household appliance according to one of claims 1 to 7, **characterised in that** the household appliance includes a container, in which a food is stored, processed or prepared.

9. Household appliance according to claim 8, **characterised in that** the household appliance is selected from the group comprising a refrigerator, a coffee machine, an oven, a microwave, a vegetable cutter and a juicer.

10. Household appliance according to one of claims 1 to 7, **characterised in that** the household appliance is a water-bearing household appliance with a container for receiving objects to be treated, which is selected from the group comprising a washing machine, a washer-dryer, a dryer and a dishwasher.

11. Household appliance according to one of claims 1 to 10, **characterised in that** the at least one inner surface is formed by particles, which contain at least one salt containing polyoxometalate or consist of the same, wherein the particles are positioned in a filter unit, which is permeable for a gaseous or liquid medium.

12. Household appliance according to claim 11, **characterised in that** the household appliance has a circulation element and/or air introduction element assigned to the filter unit.

13. Household appliance according to one of claims 1 to 12, including a means of generating or introducing an oxidising agent, the oxidising effect of which on microorganisms is enhanced by interaction with the inner or outer surface of the household appliance containing a polyoxymetalate.

14. Household appliance according to claim 13, **characterised in that** the oxidising means is oxygen, ozone or a peroxide.

15. Household appliance according to one of claims 1 to 14, **characterised in that** the polyoxometalate is present at least partially as tetra-alkylammonium salt.

## Revendications

1. Appareil domestique qui contient, dans une surface, au moins une substance catalytiquement active, **caractérisé en ce que** la substance catalytiquement active est un polyoxométallate, qui est contenu dans une surface interne et/ou externe de l'appareil domestique, à condition que le polyoxométallate soit contenu au moins dans une surface externe de l'appareil domestique lorsque l'appareil domestique est un appareil domestique alimenté en eau présentant un récipient pour recevoir des objets à nettoyer, une surface externe de l'appareil domestique étant une surface qui est accessible, lors d'un fonctionnement usuel de l'appareil domestique, pour un utilisateur de l'appareil domestique sans perturber le fonctionnement, et une surface interne de l'appareil domestique étant une surface qui n'est pas accessible, lors d'un fonctionnement usuel de l'appareil domestique, pour un utilisateur de l'appareil domestique sans perturber le fonctionnement.

2. Appareil domestique selon la revendication 1, **caractérisé en ce que** le polyoxométallate est un tungstate.

3. Appareil domestique selon la revendication 2, **caractérisé en ce que** le tungstate contient du vanadium.

4. Appareil domestique selon la revendication 3, **caractérisé en ce que** le tungstate comprend l'anion [SiV₃W₉O₄₀]⁷⁻.

5. Appareil domestique selon la revendication 1 ou 2, **caractérisé en ce que** le tungstate est modifié par du titane.

6. Appareil domestique selon l'une quelconque des revendications 1 à 5, **caractérisé en ce qu'**au moins une surface externe contenant du polyoxométallate comprend un élément de commande de l'appareil domestique.

7. Appareil domestique selon l'une quelconque des revendications 1 à 6, **caractérisé en ce qu'**une surface interne contenant du polyoxymétallate est une paroi interne d'un réservoir à eau et/ou d'un élément d'écoulement de l'appareil domestique.

8. Appareil domestique selon l'une quelconque des revendications 1 à 7, **caractérisé en ce que** l'appareil domestique comprend un récipient dans lequel un aliment est conservé, transformé ou préparé.

9. Appareil domestique selon la revendication 8, **caractérisé en ce que** l'appareil domestique est choisi dans le groupe qui est constitué par un appareil de refroidissement, une machine à café, un four, un appareil à micro-ondes, un appareil pour la découpe de légumes et un extracteur de jus.

10. Appareil domestique selon l'une quelconque des revendications 1 à 7, **caractérisé en ce que** l'appareil domestique est un appareil domestique alimenté en eau présentant un récipient pour recevoir des objets à traiter, qui est choisi dans le groupe constitué par un lave-linge, un séchoir pour le linge, un séchoir et un lave-vaisselle.

11. Appareil domestique selon l'une quelconque des revendications 1 à 10, **caractérisé en ce que** ladite au moins une surface interne est formée par des particules qui contiennent au moins un sel contenant du polyoxymétallate ou qui sont constituées par celui-ci, les particules étant placées dans une unité de filtration qui est perméable pour un milieu gazeux ou liquide.

12. Appareil domestique selon la revendication 11, **caractérisé en ce que** l'appareil domestique présente un élément de circulation et/ou un élément d'introduction d'air associé à l'unité de filtration.

13. Appareil domestique selon l'une quelconque des revendications 1 à 12, comprenant un moyen pour générer ou introduire un oxydant, dont l'effet oxydant sur des micro-organismes est renforcé par interaction avec la surface interne ou externe, contenant un polyoxymétallate, de l'appareil domestique.

14. Appareil domestique selon la revendication 13, **caractérisé en ce que** l'oxydant est l'oxygène, l'ozone ou un peroxyde.

15. Appareil domestique selon l'une quelconque des revendications 1 à 14, **caractérisé en ce que** le polyoxymétallate se trouve au moins en partie sous forme de sel de tétraalkylammonium.
